# EUROPEAN PATENT SPECIFICATION

(11) **EP 2 579 041 B1**
(45) Date of publication and mention of the grant of the patent: **13.04.2016**
(21) Application number: 11789131.7
(22) Date of filing: 20.05.2011
(51) Int. Cl.: G01N 33/558, G01N 33/68

(54) **Rapid detection tool and kit for premature rupture of fetal membrane using ICAM-1 as marker.**
Hilfsmittel und Kit zur schnellen Erkennung des vorzeitigen Risses einer Fruchtblase mit ICAM-1 als Marker.
Outil et nécessaire de détection rapide d'une rupture prématurée des membranes fétales utilisant l'ICAM-1 comme marqueur.

(30) Priority: 02.06.2010 CN 201010189683
(43) Date of publication of application: 10.04.2013
(73) Proprietor: Origissay Biologics Technology Co. Ltd, Chengdu, Sichuan 610041 (CN)
(72) Inventor: HU, Huaizhong, Sichuan 610041 (CN); GUAN, Xiangqian, Sichuan 610072 (CN); XIANG, Hongfa, Sichuan 610041 (CN); ZHOU, Rong, Sichuan 610041 (CN)
(74) Representative: Zwicker, Jörk
(86) International application number: PCT/CN2011/074383
(87) International publication number: WO 2011/150752

(56) References cited:
- WO-A2-2004/014220
- CN-A- 101 216 493
- CN-A- 101 271 110
- CN-A- 101 871 943
- CN-U- 201 697 921
- US-A- 5 656 503
- US-A1- 2007 172 963
- ZOU ET AL: "The value of the soluable intercellular adhesion molecule-1 levels in matermal serum for determination of occult chorioamnionitis in premature rupture of membranes.", JOURNAL OF HUAZHONG UNIVERSITY OF SCIENCE AND TECHNOLOGY, vol. 24, no. 2, 1 January 2004 (2004-01-01), pages 154-157, XP055080445, ISSN: 1672-0733
- MOHAMED SHAARAWY ET AL: "THE CLINICAL VALUE OF ASSAYING MATERNAL SERUM AND AMNIOTIC FLUID INTERCELLULAR ADHESION MOLECULE 1 (ICAM-1) IN CASES OF PREMATURE RUPTURE OF MEMBRANES", CYTOKINE, vol. 10, no. 12, 1 December 1998 (1998-12-01), pages 989-992, XP055080451, ISSN: 1043-4666, DOI: 10.1006/cyto.1998.0382
- TAO WANG ET AL: "Proteins in leaked amniotic fluid as biomarkers diagnostic for prelabor rupture of membranes", PROTEOMICS - CLINICAL APPLICATIONS, vol. 5, no. 7-8, 1 August 2011 (2011-08-01), pages 415-421, XP055080447, ISSN: 1862-8346, DOI: 10.1002/prca.201000123
- LIU YAN: 'The Expression and Clinical Significance of Intercellular Adhesion' MOLECULE-1 IN PREMATURE RUPTURE OF MEMBRANES. vol. 25, no. 6, June 2009, pages 357 - 359

## Description

### TECHNICAL FIELD

The present application relates to a medical detection tool, particularly to a detection tool and kit for detecting premature rupture of fetal membrane (PROM) taking ICAM-1 as detection marker.

### BACKGROUND

Rupture of fetal membrane (ROM) could happen anytime during pregnancy. The fetal membrane rupture happened before labor is known as premature rupture of fetal membrane (PROM). The incidence of PROM is about 10% after full-term (37 weeks gestation) and 2-3.5% before full-term (before 37 weeks gestation). PROM is the leading cause of prenatal and postnatal complications encountered by obstetric patients. It is responsible for premature labor and is the main reason why a newborn is received into ICU. For a patient with PROM, the medical doctor has to seek balance between prolonging gestation and the risks of intrauterine and maternal infection as well as conditions involving lung development in fetus. The management of a PROM patient can be difficult and costly and therefore timely and accurate diagnosis of PROM is of great importance.

The current procedure, which is considered as the golden standard for the diagnosis of PROM, is injecting dye (for example methylene blue) into the amniotic cavity of a patient, and the patient is diagnosed with PROM if stained fluid is observed from vagina. However, it is hard to put such procedure into clinical use because of the discomfort caused by it. The indicators normally used for the diagnosis of PROM are the medical history of a patient, the amniotic fluid outflow experienced by a patient from vagina, the alkaline pH of vaginal fluid (normal vaginal fluid has a pH of 4.5-5.5), the amniotic fluid crystallization observed in the microscopic examination of vaginal fluid smear. These indicators are easy to observe, but have low sensitivity and accuracy. For test devices for detecting analytes in biological samples see US 5,656,503. Devices and methods for detecting amniotic fluid in vaginal secretions are described in WO 2004/014220.

Intercellular adhesion molecule-1 (referred to herein as ICAM-1) is an adhesion molecule of the immunoglobulin superfamily. It is a single-chain glycoprotein with a molecular weight of 76kD-114kD. ICAM-1 is widely expressed on the surface of vascular endothelial cells, thymic epithelial cells and other non-blood derived epithelial cells, the surface of T lymphocytes, B lymphocytes and dendritic cells and other blood derived cells, and the surface of fibroblast. ICAM-1 is involved in lymphocytic infiltration. The level of soluble ICAM-1 in amniotic fluid is elevated during late pregnancy (28-40 weeks gestation) due to the ICAM-1 expression in monocytes from amniotic fluid and fetal membrane and the activation of neutrophil. Maternal serum levels of soluble ICAM and C-reactive protein (CRP) are significantyl higher in women with PROM than in those without (Zou et al., The value of soluble intercellular adhesion molecule-1 levels in maternal serum for determination of occult chorioamnionitis in premature rupture of membranes, J. of Huazhong Univ. of Science and Technology, Vol. 24, No. 2, 1 January 2004, p. 154-157). Another study determined levels of ICAM-1 in sera and amniotic fluid of cases of PROM ( see Shaarawy et al., "The clinical value of assaying maternal serum and amniotic fluid ICAM-1 in cases of RPOM", Cytokine, Vol. 10, No. 12, 1 December 1998, p. 989-992). However, currently no report discloses the rapid detection tool for premature rupture of fetal membrane taking ICAM-1 as detection marker.

### DESCRIPTION

The object of the present application is to overcome the disadvantages of the prior art, and to provide a method for rapidly detecting premature rupture of fetal membrane by using inter-cellular adhesion molecule-1 as detection marker and using vaginal fluid as a sample, which is performed by a rapid detection tool and kit for premature rupture of fetal membrane taking ICAM-1 as detection marker, by which the result is obtained rapidly with a non-invasive approach.

The present invention is defined in its broadest sense by the appended claims.

The inventor of the present application discovered by experiments that ICAM-1 is one of the major proteins existed in amniotic fluid during late pregnancy, with a content of 13.08±6.11 ng/ml. Once PROM happens, the amniotic fluid leaks from amniotic cavity to vagina, therefore the soluble ICAM-1 in amniotic fluid would appear in cervical-vaginal fluid (CVF). The screening tests conducted by protein microarray revealed that the concentration of ICAM-1 in the CVF sample of pregnant woman diagnosed with PROM is significantly different compared with the pregnant woman who did not diagnosed with PROM (both age and gestational weeks are matched between the two groups, and there is no major diseases and pregnancy complications in both groups). Therefore, ICAM-1 can be used as detection marker for PROM.

The present rapid detection tool for premature rupture of fetal membrane with ICAM-1 as detection marker comprises: a lining sheet, a water absorbing pad, a nitrocellulose membrane, a gold-label pad and a sample pad. Said water absorbing pad, nitrocellulose membrane, gold-label pad and sample pad are sequentially connected to each next one from top to bottom and bound to the lining sheet, and the gold-label pad is partly covered by the sample pad. A detection line formed by disposing sheep anti-human ICAM-1 polyclonal antibody and a control line formed by disposing sheep anti-mouse immunoglobulin G polyclonal antibody are disposed on the nitrocellulose membrane, wherein the detection line is located below the control line and spaced apart. The gold-label pad is formed by water absorbing fibers coated with a gold-label mouse anti-human ICAM-1 monoclonal antibody conjugate.

The present rapid detection kit for premature rupture of fetal membrane with ICAM-1 as detection marker is composed of a cassette and the above-mentioned detection tools contained in said cassette. The inner chamber of the cassette is adapted to the shape and size of the detection tool. An opening for the detection tool is disposed on the top wall of the cassette. A sample hole and an observation hole are disposed on the side wall of the cassette. When the detection tools is housed in the cassette, the location of the sample hole corresponds to the location of the sample pad, and the location of the observation hole corresponds to the location of the detection line and the control line.

The width of the detection line and the control line of the above detection tool is preferably 0.8 mm - 1mm, and the distance between the detection line and the control line is preferably 10mm - 12mm. The shape of the detection tool is preferably rectangle considering the cost and the ease of manufacture, however, the detection tool can have any shape suitable. The water absorbing pad is a pad made of water absorbing paper. The sample pad, i.e. water absorbing fiber pad, is a pad made of water absorbing fibers.

The production method of the present rapid detection tool for premature rupture of fetal membrane with ICAM-1 as detection marker is:

### 1. Disposing of the detection line and the control line

The method for disposing sheep anti-human ICAM-1 polyclonal antibody (R&D Inc.) and sheep anti-mouse IgG polyclonal antibody (SHANGHAI BIOENGINE LABORATORY CO., LTD.) on nitrocellulose membrane see Hou Hui-ren et al., The preparation of AFP immunochromatographic test strips, Isotope, 2000,13(3):124-126. The concentration of the operating solution of sheep anti-human ICAM-1 polyclonal antibody is 1-3 mg/ml. The concentration of the operating solution of sheep anti-mouse IgG polyclonal antibody is 1-3 mg/ml.

### 2. Preparation of gold-label pad

### (1) The preparation of colloid gold

Gold chloride solution (0.01 g/100ml) is formed by dissolving gold chloride into triple-distilled water and heated to boiling. Trisodium citrate solution (9g - 10g/100ml) is added to gold chloride solution with stirring to reach a concentration of 1.35~1.5 ml trisodium citrate solution per 100 ml gold chloride solution. The mixture is boiled to present a transparent orange color (about 7 to 10 min). Then the mixture is cooled to room temperature. The mixture is adjusted with K2CO3 solution (0.1~0.2 mol/L) to a pH of 7.0~7.2 and the colloid gold is thus obtained.

### (2) Preparation of gold-label mouse anti-human ICAM-1 monoclonal antibody conjugate

Mouse anti-human ICAM-1 monoclonal antibody (R&D Inc.) is added to the colloid gold obtained in step (1). After evenly mixed bovine serum albumin (BSA, Roche) is added thereto. After evenly mixed NaCl solution (10g/100ml) is added thereto. The concentration of mouse anti-human ICAM-1 monoclonal antibody in colloid gold solution is 200ug/100m1. The concentration of bovine serum albumin in colloid gold solution is 250mg/100ml.The concentration of NaCl solution in colloid gold solution is 1g/100ml. After each mix, the mixture is centrifuged at 2000 rpm, 4000 rpm and 10000 rpm respectively for at least 10min. The product thus obtained is gold-label mouse anti-human ICAM-1 monoclonal antibody conjugate. The conjugate is dissolved in phosphate buffer (pH 7.2) containing sodium azide (90mg/100ml), and stored at 4 °C for the latter use.

### (3) Preparation of gold-label pad

The water absorbing fibers are soaked in bovine serum albumin (BSA, Roche) solution (0.5g/100ml) under 22-25°C for at least 1hr. The gold-label mouse anti-human ICAM-1 monoclonal antibody conjugate obtained in step (2) is coated on the water absorbing fibers (for the coating method see Hou Hui-ren et al., The preparation of AFP immunochromatographic test strips, Isotope, 2000,13(3):124-126) and gold-label pad is thus obtained.

### 3. Preparation of the rapid detection tool for premature rupture of fetal membrane with ICAM-1 as detection marker

The sheet of water absorbing pad 2, the nitrocellulose membrane 5 disposed with detection line and control line, the gold-label pad 6 and the sample pad 7 are fixed on the sheet of lining sheet 1 and cut into rectangular strip.

For the detection tool and the kit used in the present invention, the test strip is effective if the control line present a clear purple line; the result is positive (i.e. with PROM) if the detection line present a purple line; the result is negative (i.e. without PROM) if the detection line present no colored line.

The detection tool and the kit used in the present invention has the following advantages:
1. The present disclosure provides a detection tool utilizing new detection marker for detecting PROM.
2. Sampled experiment conducted by the kit used in the present invention on pregnant woman diagnosed as with or without PROM shows that the sensitivity of the present kit is around 96%, and the specificity of the present kit is around 91%.
3. The kit is easy to use and it takes 10-15 min to examine one sample. The detection speed is significantly faster compared with the available clinical detection methods conventionally used.
4. It is non-invasive since the sample used for detection is vaginal fluid.
5. The detection tool and the kit used in the present invention has simple structure and low cost, and is easy to make and suitable for industrial production.

The detection tool and the kit used in the present invention is suitable for all pregnant woman, especially suitable for the pregnant woman with the risk of premature labor or abdominal injury.

### DESCRIPTION OF THE DRAWINGS

Fig. 1 is a schematic diagram of a rapid detection tool of the present invention for premature rupture of fetal membrane with ICAM-1 as detection marker.
Fig. 2 is the left view of Fig. 1
Fig. 3 is a schematic diagram of a cassette of a rapid detection kit of the present invention for premature rupture of fetal membrane with ICAM-1 as detection marker.
Fig. 4 is the top view of Fig. 3.
Fig. 5 is the sectional view taken along A-A of Fig. 3.
Fig. 6 is a schematic diagram of a rapid detection kit of the present invention for premature rupture of fetal membrane with ICAM-1 as detection marker.

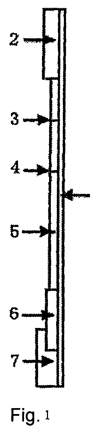

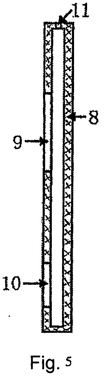

In Fig. 1-6, 1-lining sheet, 2-water absorbing pad, 3-control line, 4-detection line, 5-nitrocellulose membrane, 6-gold-label pad, 7-sample pad, 8-cassette, 9-observation hole, 10-sample hole, 11-opening for detection tool, 12-detection tool, 13-ICAM-1 in tested sample.

### EXAMPLE

The structure, production method and use of the present rapid detection tool and kit for premature rupture of fetal membrane with ICAM-1 as detection marker is further illustrated with reference to the drawings.

### Example 1

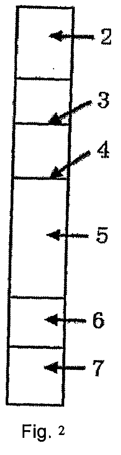

In this example, the rapid detection tool for premature rupture of fetal membrane with ICAM-1 as detection marker (see Fig. 1 and 2) has a rectangle shape and comprises: lining sheet 1, water absorbing pad 2, a nitrocellulose membrane 5, gold-label pad 6 and sample pad 7. Said water absorbing pad, nitrocellulose membrane, gold-label pad and sample pad are sequentially connected to each next one from top to bottom and bound to the lining sheet, and the gold-label pad is partly covered by the sample pad. Lining 1 is made of high whiteness medical plastic PVC sheet coated with pressure sensitive adhesive and is inert to biologically active molecules. Water absorbing pad 2 is made of water absorbing paper. A detection line 4 formed by disposing sheep anti-human ICAM-1 polyclonal antibody and a control line 3 formed by disposing sheep anti-mouse immunoglobulin G polyclonal antibody are disposed on the nitrocellulose membrane 5, wherein the detection line is located below the control line. The width of the detection line and the control line is about 1 mm (or 0.8 mm or 0.9 mm), and the distance between the detection line and the control line is 10 mm (or 11 mm or 12 mm). The gold-label pad 6 is formed by glass fiber film or polyester fiber membrane coated with a gold-label mouse anti-human ICAM-1 monoclonal antibody conjugate. The sample pad is a pad made from glass fiber film or polyester fiber membrane.

In this example, the production method of the rapid detection tool for premature rupture of fetal membrane with ICAM-1 as detection marker is:

### 1. The disposing of the detection line and the control line

The method for disposing sheep anti-human ICAM-1 polyclonal antibody (R&D Inc.) and sheep anti-mouse IgG polyclonal antibody (SHANGHAI BIOENGINE LABORATORY CO.,LTD.) on nitrocellulose membrane see Hou Hui-ren et al., The preparation of AFP immunochromatographic test strips, Isotope, 2000,13(3):124-126.

### 2. The preparation of gold-label pad

### (1) The preparation of colloid gold

Gold chloride solution is formed by dissolving 0.01g gold chloride into 100ml triple-distilled water and 100ml gold chloride solution is heated to boiling. 1.5ml trisodium citrate solution (10g/100ml) is added with stirring. The mixture is boiled to present a transparent orange color (about 7 to 10 min). After cooled to room temperature, the mixture is adjusted with K₂CO₃ solution to a pH of 7.0~7.2 and the colloid gold is thus obtained.

### (2) The preparation of gold-label mouse anti-human ICAM-1 monoclonal antibody conjugate

200ug mouse anti-human ICAM-1 monoclonal antibody (R&D Inc.) is added to 100ml colloid gold obtained in step (1). After evenly mixed 250 mg bovine serum albumin (BSA, Roche) is added thereto. After evenly mixed NaCl solution (10g/100ml) is added thereto. The concentration of NaCl solution in colloid gold solution is 1g/100ml. After each mix, the mixture is centrifuged at 2000 rpm, 4000 rpm and 10000 rpm respectively for 10min. The product thus obtained is gold-label mouse anti-human ICAM-1 monoclonal antibody conjugate. The conjugate is dissolved in phosphate buffer (pH 7.2) containing sodium azide (90mg/100ml), and stored at 4 °C for latter use.

### (3) The preparation of gold-label pad

The glass fiber film or polyester fiber membrane is soaked in bovine serum albumin (BSA, Roche) solution (0.5 g/100ml) under 22-25°C for at least 1hr. The gold-label mouse anti-human ICAM-1 monoclonal antibody conjugate obtained in step (2) is coated on the glass fiber film or polyester fiber membrane (for the coating method see Hou Hui-ren et al., The preparation of AFP immunochromatographic test strips, Isotope, 2000,13(3):124-126) and gold-label pad is thus obtained.

### 3. The preparation of the rapid detection tool for premature rupture of fetal membrane with ICAM-1 as detection marker

The sheet of water absorbing pad 2, the nitrocellulose membrane 5 disposed with detection line and control line, the gold-label pad 6 and the sample pad 7 are fixed on the sheet of lining sheet 1 and cut into rectangular strip.

### Example 2

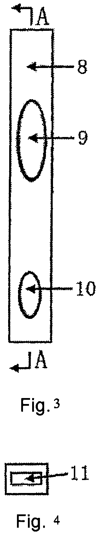

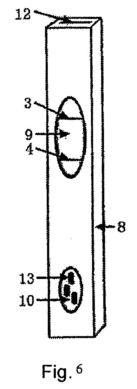

In this example, the rapid detection kit with the shape and construction shown in Fig. 6 for premature rupture of fetal membrane taking ICAM-1 as detection marker is composed of a cassette 8 and the detection tool 12 contained in said cassette. The detection tool 12 has the shape and construction shown the Example 1. The cassette is made of medical plastics and has rectangle shape (as shown in Fig. 3, 4 and 5). The inner chamber of the cassette is adapted to the shape and size of the detection tool. An opening 11 for the detection tool is disposed on the top wall of the cassette. A sample hole 10 and an observation hole 9 are disposed on the side wall of the cassette. When the detection tool is housed in the cassette, the location of the sample hole 10 corresponds to the location of the sample pad 7, and the location of the observation hole 9 corresponds to the location of the detection line 4 and the control line 3. The kit is sealed in aluminum foil bag containing drier for later use.

The mechanism and the use of the kit are as follows:
The sample to be tested (cervical-vaginal fluid of pregnant woman) is loaded onto sample pad 7 through the sample hole 10 on cassette 8. Said sample moves chromatographically from sample pad 7 along the detection strip up to gold labeled pad 6. The sample dissolves the gold-label mouse anti-human ICAM-1 monoclonal antibody conjugate and moves further up to the detection line 4 on nitrocellulose membrane. If ICAM-1 is present in said sample, the sheep anti-human ICAM-1 polyclonal antibody on detection line 4 and gold-label mouse anti-human ICAM-1 monoclonal antibody as well as the ICMA-1 antigen on the sample will form purple sandwich immune complex, and this clear purple line on the detection line indicates a positive result. The more ICAM-1 antigen in the sample, the darker the color is. If there is no ICAM-1 antigen in the sample, the gold-label mouse anti-human ICAM-1 monoclonal antibody will not form complex with the sheep anti-human ICAM-1 polyclonal antibody on detection line 4, therefore no colored line is formed on detection line and the result is negative. The sample and the gold-label mouse anti-human ICAM-1 monoclonal antibody moves up to control line 3 and the sheep anti-mouse IgG polyclonal antibody on the control line will combine with the gold-label mouse anti-human ICAM-1 monoclonal antibody and form a red or purple line on the control line, which indicates the detection tool is effective. The result can be visualized through the observation hole 9 on the cassette.

The present rapid detection tool and kit for premature rupture of fetal membrane with ICAM-1 as detection marker is described with reference to the examples.

## Claims

1. A method for rapidly detecting premature rupture of fetal membrane by using inter-cellular adhesion molecule-1 as detection marker and using vaginal fluid as a sample which is performed by a rapid detection tool comprising: a lining sheet (1), a water absorbing pad (2), a nitrocellulose membrane (5), a gold-label pad (6) and a sample pad (7), wherein said water absorbing pad (2), nitrocellulose membrane (5), gold-label pad (6) and sample pad (7) are sequentially connected to each next one from top to bottom and bound to the lining sheet (1), and the gold-label pad (6) is partly covered by the sample pad;
a detection line (4) formed by coating sheep anti-human inter-cellular adhesion molecule-1 polyclonal antibody and a control line (3) formed by coating sheep anti-mouse immunoglobulin G polyclonal antibody are disposed on the nitrocellulose membrane (5), wherein the detection line is located below the control line, and the detection line and the control line are spaced apart;
the gold-label pad (6) is formed by water absorbing fibers coated with a gold-label mouse anti-human inter-cellular adhesion molecule-1 monoclonal antibody conjugate.

2. The method according to claim 1, wherein the width of the detection line (4) and the control line (3) is 0.8 mm - 1mm.

3. The method according to claim 1 or 2, wherein the distance between the detection line (4) and the control line (3) is 10mm - 12mm.

4. The method according to claim 1 or 2, wherein the rapid detection tool is rectanglular.

5. The method according to claim 3, wherein the rapid detection tool is rectangluar.

6. The method according to claim 1, which is performed by a rapid detection kit, which is composed of a cassette (8) and a detection tool contained in said cassette, wherein said detection tool comprises: a lining sheet (1), a water absorbing pad (2), a nitrocellulose membrane (5), a gold-label pad (6) and a sample pad (7). Said water absorbing pad (2), nitrocellulose membrane (5), gold-label pad (6) and sample pad (7) are sequentially connected to each next one from top to bottom and bound to the lining sheet (1), and the gold-label pad (6) is partly covered by the sample pad; a detection line (4) formed by coating sheep anti-human inter-cellular adhesion molecule-1 polyclonal antibody and a control line (3) formed by coating sheep anti-mouse immunoglobulin G polyclonal antibody are disposed on the nitrocellulose membrane (5), the detection line is located below the control line, and the detection line and the control line are spaced apart; the gold-label pad (6) is formed by water absorbing fibers coated with a gold-label mouse anti-human inter-cellular adhesion molecule-1 monoclonal antibody conjugate;
the inner chamber of the cassette is adapted to the shape and size of the detection tool, an opening (11) for inserting the detection tool is disposed on the top wall of the cassette, a sample hole (10) and an observation hole (9) are disposed on the side wall of the cassette, when the detection tool is housed in the cassette, the location of the sample hole (10) corresponds to the location of the sample pad (7), and the location of the observation hole (9) corresponds to the location of the detection line (4) and the control line (3).

7. The method according to claim 6, wherein the detection tool is rectanglular.

8. Use of a rapid detection tool in rapid detection of premature rupture of fetal membrane by using inter-cellular adhesion molecule-1 as detection marker and using vaginal fluid as a sample, wherein the rapid detection tool comprises: a lining sheet (1), a water absorbing pad (2), a nitrocellulose membrane (5), a gold-label pad (6) and a sample pad (7), wherein said water absorbing pad (2), nitrocellulose membrane (5), gold-label pad (6) and sample pad (7) are sequentially connected to each next one from top to bottom and bound to the lining sheet (1), and the gold-label pad (6) is partly covered by the sample pad; a detection line (4) formed by coating sheep anti-human inter-cellular adhesion molecule-1 polyclonal antibody and a control line (3) formed by coating sheep anti-mouse immunoglobulin G polyclonal antibody are disposed on the nitrocellulose membrane (5), wherein the detection line is located below the control line, and the detection line and the control line are spaced apart;
the gold-label pad (6) is formed by water absorbing fibers coated with a gold-label mouse anti-human inter-cellular adhesion molecule-1 monoclonal antibody conjugate.

9. The use according to claim 8, wherein the width of the detection line (4) and the control line (3) is 0.8 mm- 1mm.

10. The use according to claim 8 or 9, wherein the distance between the detection line (4) and the control line (3) is 10mm - 12mm.

11. The use according to claim 8 or 9, wherein the rapid detection tool is rectanglular.

12. The use according to claim 10, wherein the rapid detection tool is rectanglular.

13. Use of a rapid detection kit in rapid detection of premature rupture of fetal membrane wherein inter-cellular adhesion molecule-1 is used as detection marker and vaginal fluid is used as a sample, and wherein the rapid detection kit is composed of a cassette (8) and a detection tool contained in said cassette,
wherein said detection tool comprises: a lining sheet (1), a water absorbing pad (2), a nitrocellulose membrane (5), a gold-label pad (6) and a sample pad (7). Said water absorbing pad (2), nitrocellulose membrane (5), gold-label pad (6) and sample pad (7) are sequentially connected to each next one from top to bottom and bound to the lining sheet (1), and the gold-label pad (6) is partly covered by the sample pad; a detection line (4) formed by coating sheep anti-human inter-cellular adhesion molecule-1 polyclonal antibody and a control line (3) formed by coating sheep anti-mouse immunoglobulin G polyclonal antibody are disposed on the nitrocellulose membrane (5), the detection line is located below the control line, and the detection line and the control line are spaced apart; the gold-label pad (6) is formed by water absorbing fibers coated with a gold-label mouse anti-human inter-cellular adhesion molecule-1 monoclonal antibody conjugate;
the inner chamber of the cassette is adapted to the shape and size of the detection tool, an opening (11) for inserting the detection tool is disposed on the top wall of the cassette, a sample hole (10) and an observation hole (9) are disposed on the side wall of the cassette, when the detection tool is housed in the cassette, the location of the sample hole (10) corresponds to the location of the sample pad (7), and the location of the observation hole (9) corresponds to the location of the detection line (4) and the control line (3).

## Patentansprüche

1. Verfahren zum schnellen Nachweisen einer verfrühten Ruptur einer Fetalmembran unter Verwendung eines interzellulären Adhäsionsmoleküls-1 als Nachweismarker und unter Verwendung einer vaginalen Flüssigkeit als einer Probe, das durch ein schnelles Nachweismittel durchgeführt wird, umfassend:
eine Beschichtung (1), ein Wasser absorbierendes Pad (2), eine Nitrozellulose-Membran (5), ein mit Gold markiertes Pad (6) und ein Probenpad (7), wobei das Wasser absorbierende Pad (2), die Nitrozellulose-Membran (5), das mit Gold markierte Pad (6) und das Probenpad (7) der Reihe nach von oben nach unten mit dem jeweils nächsten verbunden sind und an die Beschichtung (1) gebunden sind, und das mit Gold markierte Pad (6) teilweise von dem Probenpad bedeckt ist;
eine Nachweislinie (4), die durch Beschichten mit einem polyklonalen Schaf-Antikörper gegen humanes interzelluläres Adhäsionsmolekül-1 gebildet ist, und eine Kontrolllinie (3), die durch Beschichten mit einem polyklonalen Schaf-Antikörper gegen Maus Immunglobulin G gebildet ist, sind auf der Nitrozellulose-Membran (5) angebracht, wobei die Nachweislinie unterhalb der Kontrolllinie angeordnet ist, und die Nachweislinie und die Kontrolllinie räumlich voneinander getrennt sind;
das mit Gold markierte Pad (6) ist durch Wasser absorbierende Fasern gebildet, die mit einem mit Gold markierten monoklonalen Maus-Antikörperkonjugat gegen humanes interzelluläres Adhäsionsmolekül-1 beschichtet sind.

2. Verfahren gemäß Anspruch 1, wobei die Breite der Nachweislinie (4) und der Kontrolllinie (3) 0,8 mm - 1 mm beträgt.

3. Verfahren gemäß Anspruch 1 oder 2, wobei der Abstand zwischen der Nachweislinie (4) und der Kontrolllinie (3) 10 mm - 12 mm beträgt.

4. Verfahren gemäß Anspruch 1 oder 2, wobei das schnelle Nachweismittel rechteckig ist.

5. Verfahren gemäß Anspruch 3, wobei das schnelle Nachweismittel rechteckig ist.

6. Verfahren gemäß Anspruch 1, das durch ein schnelles Nachweiskit durchgeführt wird, welches aus einer Kassette (8) und einem Nachweismittel, das in der Kassette enthalten ist, zusammengesetzt ist, wobei das Nachweismittel umfasst:
eine Beschichtung (1), ein Wasser absorbierendes Pad (2), eine Nitrozellulose-Membran (5), ein mit Gold markiertes Pad (6) und ein Probenpad (7);
das Wasser absorbierende Pad (2), die Nitrozellulose-Membran (5), das mit Gold markierte Pad (6) und das Probenpad (7) sind der Reihe nach von oben nach unten mit dem jeweils nächsten verbunden und an die Beschichtung (1) gebunden, und das mit Gold markierte Pad (6) ist teilweise von dem Probenpad bedeckt;
eine Nachweislinie (4), die durch Beschichten mit einem polyklonalen Schaf-Antikörper gegen humanes interzelluläres Adhäsionsmolekül-1 gebildet ist, und eine Kontrolllinie (3), die durch Beschichten mit einem polyklonalen Schaf-Antikörper gegen Maus Immunglobulin G gebildet ist, sind auf der Nitrozellulose-Membran (5) angebracht, wobei die Nachweislinie unterhalb der Kontrolllinie angeordnet ist, und die Nachweislinie und die Kontrolllinie räumlich voneinander getrennt sind;
das mit Gold markierte Pad (6) ist durch Wasser absorbierende Fasern gebildet, die mit einem mit Gold markierten monoklonalen Maus-Antikörperkonjugat gegen humanes interzelluläres Adhäsionsmolekül-1 beschichtet sind;
die innere Kammer der Kassette ist an die Form und Größe des Nachweismittels angepasst, eine Öffnung (11) zum Einfügen des Nachweismittels ist an der Oberseite der Kassette angebracht, eine Probenöffnung (10) und eine Beobachtungsöffnung (9) sind an der Seitenwand der Kassette angebracht, wenn sich das Nachweismittel in der Kassette befindet, entspricht die Position der Probenöffnung (10) der Position des Probenpads (7), und die Position der Beobachtungsöffnung (9) entspricht der Position der Nachweislinie (4) und der Kontrolllinie (3).

7. Verfahren gemäß Anspruch 6, wobei das Nachweismittel rechteckig ist.

8. Verwendung eines schnellen Nachweismittels zum schnellen Nachweis einer verfrühten Ruptur einer Fetalmembran unter Verwendung eines interzellulären Adhäsionsmoleküls-1 als Nachweismarker und unter Verwendung einer vaginalen Flüssigkeit als einer Probe, wobei das schnelle Nachweismittel umfasst:
eine Beschichtung (1), ein Wasser absorbierendes Pad (2), eine Nitrozellulose-Membran (5), ein mit Gold markiertes Pad (6) und ein Probenpad (7),
wobei das Wasser absorbierende Pad (2), die Nitrozellulose-Membran (5), das mit Gold markierte Pad (6) und das Probenpad (7) der Reihe nach von oben nach unten mit dem jeweils nächsten verbunden sind und an die Beschichtung (1) gebunden sind, und das mit Gold markierte Pad (6) teilweise von dem Probenpad bedeckt ist;
eine Nachweislinie (4), die durch Beschichten mit einem polyklonalen Schaf-Antikörper gegen humanes interzelluläres Adhäsionsmolekül-1 gebildet ist, und eine Kontrolllinie (3), die durch Beschichten mit einem polyklonalen Schaf- Antikörper gegen Maus Immunglobulin G gebildet ist, sind auf der Nitrozellulose-Membran (5) angebracht, wobei die Nachweislinie unterhalb der Kontrolllinie angeordnet ist, und die Nachweislinie und die Kontrolllinie räumlich voneinander getrennt sind;
das mit Gold markierte Pad (6) ist durch Wasser absorbierende Fasern gebildet, die mit einem mit Gold markierten monoklonalen Maus-Antikörperkonjugat gegen humanes interzelluläres Adhäsionsmolekül-1 beschichtet sind.

9. Verwendung gemäß Anspruch 8, wobei die Breite der Nachweislinie (4) und der Kontrolllinie (3) 0,8 mm - 1 mm beträgt.

10. Verwendung gemäß Anspruch 8 oder 9, wobei der Abstand zwischen der Nachweislinie (4) und der Kontrolllinie (3) 10 mm - 12 mm beträgt.

11. Verwendung gemäß Anspruch 8 oder 9, wobei das schnelle Nachweismittel rechteckig ist.

12. Verwendung gemäß Anspruch 10, wobei das schnelle Nachweismittel rechteckig ist.

13. Verwendung eines schnellen Nachweiskits zum schnellen Nachweis einer verfrühten Ruptur einer Fetalmembran, wobei ein interzelluläres Adhäsionsmoleküls-1 als Nachweismarker verwendet wird und eine vaginalen Flüssigkeit als eine Probe verwendet wird, und
wobei das schnelle Nachweiskit aus einer Kassette (8) und einem Nachweismittel, das in der Kassette enthalten ist, zusammengesetzt ist, wobei das Nachweismittel umfasst:
eine Beschichtung (1), ein Wasser absorbierendes Pad (2), eine Nitrozellulose-Membran (5), ein mit Gold markiertes Pad (6) und ein Probenpad (7);
das Wasser absorbierende Pad (2), die Nitrozellulose-Membran (5), das mit Gold markierte Pad (6) und das Probenpad (7) sind der Reihe nach von oben nach unten mit dem jeweils nächsten verbunden und an die Beschichtung (1) gebunden, und das mit Gold markierte Pad (6) ist teilweise von dem Probenpad bedeckt;
eine Nachweislinie (4), die durch Beschichten mit einem polyklonalen Schaf-Antikörper gegen humanes interzelluläres Adhäsionsmolekül-1 gebildet ist, und eine Kontrolllinie (3), die durch Beschichten mit einem polyklonalen Schaf-Antikörper gegen Maus Immunglobulin G gebildet ist, sind auf der Nitrozellulose-Membran (5) angebracht, wobei die Nachweislinie unterhalb der Kontrolllinie angeordnet ist, und die Nachweislinie und die Kontrolllinie räumlich voneinander getrennt sind;
das mit Gold markierte Pad (6) ist durch Wasser absorbierende Fasern gebildet, die mit einem mit Gold markierten monoklonalen Maus-Antikörperkonjugat gegen humanes interzelluläres Adhäsionsmolekül-1 beschichtet sind;
die innere Kammer der Kassette ist an die Form und Größe des Nachweismittels angepasst, eine Öffnung (11) zum Einfügen des Nachweismittels ist an der Oberseite der Kassette angebracht, eine Probenöffnung (10) und eine Beobachtungsöffnung (9) sind an der Seitenwand der Kassette angebracht, wenn sich das Nachweismittel in der Kassette befindet, entspricht die Position der Probenöffnung (10) der Position des Probenpads (7), und die Position der Beobachtungsöffnung (9) entspricht der Position der Nachweislinie (4) und der Kontrolllinie (3).

## Revendications

1. Procédé pour la détection rapide d'une rupture prématurée de membrane foetale comprenant l'utilisation de molécule-1 d'adhésion inter-cellulaire comme marqueur de détection et l'utilisation de fluide vaginal comme échantillon qui est traité par un dispositif de détection rapide comprenant: une feuille support (1), un tampon absorbant l'eau (2), une membrane de nitrocellulose (5), un tampon marqué à l'or (6) et un tampon pour échantillon (7), tandis que lesdits tampon absorbant l'eau (2), membrane de nitrocellulose (5), tampon marqué à l'or (6) et tampon pour échantillon (7) sont reliés séquentiellement chacun au suivant du haut vers le bas et liés à la feuille support (1), et le tampon marqué à l'or (6) est partiellement recouvert par le tampon pour échantillon;
une ligne de détection (4) formée par un revêtement d'anticorps polyclonal de molécule-1 d'adhésion inter-cellulaire anti-humain de mouton et une ligne témoin (3) formée par un revêtement d'anticorps polyclonal d'immunoglobuline G anti-souris de mouton sont disposées sur la membrane de nitrocellulose (5), tandis que la ligne de détection est située en dessous de la ligne témoin, et la ligne de détection et la ligne témoin sont espacées;
le tampon marqué à l'or (6) est formé par des fibres absorbant l'eau, revêtues avec un conjugué d'anticorps monoclonal de molécule-1 d'adhésion inter-cellulaire anti-humain de souris marqué à l'or.

2. Procédé selon la revendication 1, dans lequel la largeur de la ligne de détection (4) et de la ligne témoin (3) est de 0,8 mm - 1 mm.

3. Procédé selon la revendication 1 ou 2, dans lequel la distance entre la ligne de détection (4) et la ligne témoin (3) est de 10 mm - 12 mm.

4. Procédé selon la revendication 1 ou 2, dans lequel le dispositif de détection rapide est rectangulaire.

5. Procédé selon la revendication 3, dans lequel le dispositif de détection rapide est rectangulaire.

6. Procédé selon la revendication 1, qui est mis en oeuvre à l'aide d'un kit de détection rapide, qui est composé d'une cassette (8) et d'un dispositif de détection contenu dans ladite cassette, tandis que ledit dispositif de détection comprend: une feuille support (1), un tampon absorbant l'eau (2), une membrane de nitrocellulose (5), un tampon marqué à l'or (6) et un tampon pour échantillon (7). Lesdits tampon absorbant l'eau (2), membrane de nitrocellulose (5), tampon marqué à l'or (6) et tampon pour échantillon (7) sont reliés séquentiellement chacun au suivant du haut vers le bas et reliés à la feuille support (1), et le tampon marqué à l'or (6) est partiellement recouvert par le tampon pour échantillon; une ligne de détection (4) formée par le revêtement d'un anticorps polyclonal de la molécule-1 d'adhésion inter-cellulaire anti-humain de mouton et une ligne témoin (3) formée par le revêtement d'anticorps polyclonal d'immunoglobuline G anti-souris de mouton sont déposées sur la membrane de nitrocellulose (5), la ligne de détection est située en dessous de la ligne témoin, et la ligne de détection et la ligne témoin sont espacées; le tampon marqué à l'or (6) est formé par des fibres absorbant l'eau, revêtues avec un conjugué d'anticorps monoclonal de molécule-1 d'adhésion inter-cellulaire anti-humain de souris marqué à l'or;
la chambre intérieure de la cassette est adaptée à la forme et à la taille du dispositif de détection, une ouverture (11) pour l'insertion du dispositif de détection est disposée sur la paroi supérieure de la cassette, un orifice pour échantillon (10) et un orifice d'observation (9) sont disposés sur la paroi latérale de la cassette, lorsque le dispositif de détection est hébergé dans la cassette, la position de l'orifice pour échantillon (10) correspond à la position du tampon pour échantillon (7), et la position de l'orifice d'observation (9) correspond à la position de la ligne de détection (4) et de la ligne témoin (3).

7. Procédé selon la revendication 6, dans lequel le dispositif de détection est rectangulaire.

8. Utilisation d'un dispositif de détection rapide dans la détection rapide d'une rupture prématurée de la membrane foetale avec utilisation de molécule-1 d'adhésion inter-cellulaire comme marqueur de détection et utilisation de fluide vaginal comme échantillon, tandis que le dispositif de détection rapide comprend: une feuille support (1), un tampon absorbant l'eau (2), une membrane de nitrocellulose (5), un tampon marqué à l'or (6) et un tampon pour échantillon (7), tandis que lesdits tampon absorbant l'eau (2), membrane de nitrocellulose (5), tampon marqué à l'or (6) et tampon pour échantillon (7) sont reliés séquentiellement chacun au suivant du haut vers le bas et liés à la feuille support (1), et le tampon marqué à l'or (6) est partiellement recouvert par le tampon pour échantillon;
une ligne de détection (4) formée par le revêtement d'anticorps polyclonal de molécule-1 d'adhésion inter-cellulaire anti-humain de mouton et une ligne témoin (3) formée par le revêtement d'anticorps polyclonal d'immunoglobuline G anti-souris de mouton sont disposées sur la membrane de nitrocellulose (5), tandis que la ligne de détection est située en dessous de la ligne témoin, et que la ligne de détection et la ligne témoin sont espacées; le tampon marqué à l'or (6) est formé de fibres absorbant l'eau, revêtues avec un conjugué d'anticorps monoclonal de molécule-1 d'adhésion inter-cellulaire anti-humain de souris marqué à l'or.

9. Utilisation selon la revendication 8, dans laquelle la largeur de la ligne de détection (4) et de la ligne témoin (3) est de 0,8 mm - 1 mm.

10. Utilisation selon la revendication 8 ou 9, dans laquelle la distance entre la ligne de détection (4) et la ligne témoin (3) est de 10 mm - 12 mm.

11. Procédé selon la revendication 8 ou 9, dans lequel le dispositif de détection rapide est rectangulaire.

12. Procédé selon la revendication 10, dans lequel le dispositif de détection rapide est rectangulaire.

13. Utilisation d'un kit de détection rapide dans la détection rapide d'une rupture prématurée de membrane foetale, dans laquelle une molécule-1 d'adhésion inter-cellulaire est utilisée comme marqueur de détection et le fluide vaginal est utilisé comme échantillon, et dans laquelle le kit de détection rapide est composé d'une cassette (8) et d'un dispositif de détection contenu dans ladite cassette,
tandis que ledit dispositif de détection comprend: une feuille support (1), un tampon absorbant l'eau (2), une membrane de nitrocellulose (5), un tampon marqué à l'or (6) et un tampon pour échantillon (7). Lesdits tampon absorbant l'eau (2), membrane de nitrocellulose (5), tampon marqué à l'or (6) et tampon pour échantillon (7) sont reliés séquentiellement chacun au suivant du haut vers le bas et reliés à la feuille support (1), et le tampon marqué à l'or (6) est partiellement recouvert par le tampon pour échantillon; une ligne de détection (4) formée par le revêtement d'un anticorps polyclonal de la molécule-1 d'adhésion inter-cellulaire anti-humain de mouton et une ligne témoin (3) formée par le revêtement d'anticorps polyclonal d'immunoglobuline G anti-souris de mouton sont disposées sur la membrane de nitrocellulose (5), la ligne de détection est située en dessous de la ligne témoin, et la ligne de détection et la ligne témoin sont espacées; le tampon marqué à l'or (6) est formé par des fibres absorbant l'eau, revêtues avec un conjugué d'anticorps monoclonal de molécule-1 d'adhésion inter-cellulaire anti-humain de souris marqué à l'or;
la chambre intérieure de la cassette est adaptée à la forme et à la taille du dispositif de détection, une ouverture (11) pour l'insertion du dispositif de détection est disposée sur la paroi supérieure de la cassette, un orifice pour échantillon (10) et un orifice d'observation (9) sont disposés sur la paroi latérale de la cassette, lorsque le dispositif de détection est hébergé dans la cassette, la position de l'orifice pour échantillon (10) correspond à la position du tampon pour échantillon (7), et la position de l'orifice d'observation (9) correspond à la position de la ligne de détection (4) et de la ligne témoin (3).
